# EUROPEAN PATENT SPECIFICATION

(11) **EP 3 796 904 B1**
(45) Date of publication and mention of the grant of the patent: **25.01.2023**
(21) Application number: 18939843.1
(22) Date of filing: 16.11.2018
(51) Int. Cl.: A61K 9/16, A61K 31/405, A61K 9/48, A61P 29/00

(54) **SUSTAINED RELEASE ACEMETACIN COMPOSITIONS**
ACEMETACIN-ZUSAMMENSETZUNGEN MIT VERZÖGERTER FREISETZUNG
COMPOSITIONS D'ACÉMÉTACINE À LIBÉRATION PROLONGÉE

(43) Date of publication of application: 31.03.2021
(73) Proprietor: Santa Farma Ilaç Sanayi A.S., 34382 Istanbul (TR)
(72) Inventor: YILDIRIM, Ersin, 41455 Dilovas Kocaeli (TR); KANIK, Bayram, 41455 Dilovas Kocaeli (TR); AKTAS, Tansel, 41455 Dilovas Kocaeli (TR); ÖZTÜRK, Fatma, 41455 Dilovas Kocaeli (TR); ZOR, Ali, 41455 Dilovas Kocaeli (TR)
(74) Representative: Bulut, Pinar
(86) International application number: PCT/TR2018/050696
(87) International publication number: WO 2020/101584

(56) References cited:
- US-A- 4 900 557
- Anonymous: "EUDRAGIT@ Acrylic Polymers for Solid Oral Dosage Forms", Evonic Industries , 1 February 2017 (2017-02-01), pages 1-16, XP055707761, Retrieved from the Internet: URL:https://healthcare.evonik.com/sites/li sts/NC/DocumentsHC/Evonik-Eudragit_brochur e.pdf

## Description

### FIELD OF INVENTION

The present invention relates to sustained release pharmaceutical compositions for oral administration comprising acemetacin or a pharmaceutically acceptable salt thereof.

### STATE OF ART

Acemetacin or 0-((1-p-Chlorobenzoyl-5-methoxy-2-methylindol-3-yl)acetyl)glycolic acid is the ester of indomethacin and glycolic acidas shown Formula I. Its CAS number is 53164-05-9. Molecular weight of acemetacin is 415.8 g/mole. Molecular formula is C₂ᵢt½ClN0₆.

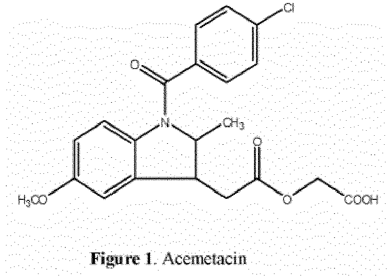

According to the British Pharmacopeia (2009), acemetacin is practically insoluble in water at acidic pH, whereas it is soluble in acetone and slightly soluble in anhydrous ethanol.

Acemetacin is first disclosed in German Patent numbered as 223465 and is a glycolic acid ester prodrug of indomethacin thereof, the efficacy of acemetacin is similar to indomethacin in the treatment of inflammatory and rheumatic disorders.

Acemetacin metabolizes to a large extent to indomethacin in metabolism. Its action profile largely corresponds to that of indomethacin. A major difference, however, is the better compatibility, since acemetacin unlike indomethacin should have only weak ulcerogenic properties. Indomethacin represents such an active ingredient in which the absorption kinetics must always be set in relation to the gastrointestinal residence time of the preparation. The maximal plasma concentrations reach in 2 -2.5 hours after dose is administered. However, bioavailability is exhibited 60% for single dose and only it reaches to 100% after multiple dosing.

Retarded acetamine has been developed by MEDA Pharmaceuticals Company under the tradename Rantudil^{®} Retard that contains 90 mg acemetacin in capsule dosage form. Its recommended dose is 90 mg/day depending on the severity and nature of disease the dosing it can be increased to 180 mg/day in divided doses. For modified release of acemetacin is absorbed from the gastrointestinal tract with maximal plasma concentrations reach over 4 - 6 hours.

Sustained release formulations are highly estimated by patients as well as by physicians since they allow the possibility of reducing dosage regimens for drugs, especially in case of an oral administration. The advantages of reduced dosage regimens for the patients are an increased convenience, better assurance of compliance, reduction of severity and frequency of side effects, since prolonged release formulations maintain substantially constant blood levels and avoid the fluctuations associated with the conventional immediate release formulations administered more than once a day.

Acemetacin in sustained release form has the advantage of all the properties mentioned above such as minimizing side effects (ulcerogenic properties), prolongs the therapeutic effect of acemetacin (from 2-2.5 hours to 4-6 hours) which leads increasing of patience compliance.

In the state of art there are many patents/patent applications which are summarized below.

EP0265858 discloses pharmaceutical composition containing acemetacin as the active ingredient in amount range between 0.1 to 5% by weight which is the topical administration for treating of acute inflammation of the joints in rheumathoid arthritis, low back pain and post-surgical pain. The present invention is not in topical dosage form thus it is not related with the invention.

EP0173210 (US4900557A) discloses sustained-release formulation containing acemetacin based on encapsulated multiple unit doses in the form of pellets containing of an initial dose which contains 10 to 60% by weight of the total amount of acemetacin and if appropriate additives, and a disintegrating agent, a binder and a substance having a relatively high specific gravity wherein pellets are coated with a lacquer which is resistant to gastric juice, the individual pellets having a diameter of 0.2 to 1.8 mm. The present invention is not in pellet form.

CN101125129 discloses a sustained release pharmaceutical composition containing acemetacin as the active ingredient wherein the sustained release layer is provided by using a mixture of xanthan gum and chitosan. The present invention does not contain xanthan gum or chitosan.

WO2007014476 discloses modified release pharmaceutical compositions containg at least one of the active ingredients indomethacin or acemetacin in micronized form (at least 50% by volume of the active ingredient has a particle size of less than 5 pm). The particle size of active pharmaceutical ingredient (API) in present invention is not in micronized form.

In accordance with the prior arts, there is a need for sustained release formulation of acemetacin wherein sustained release effect is achieved with at least two pH dependent polymers wherein acemetacin is not in micronized form.

### Summary of the Invention

The object of this invention is to develop a sustained-release oral pharmaceutical formulation comprising acemetacin or a pharmaceutically acceptable salt thereof, which has non-steroidal drug with anti-inflammatory, an analgesic and antipyretic properties, to provide additional advantages to the relevant technique.

It is a further object of the present invention relates to an oral dosage form in the form of capsule comprising acemetacin or one of its pharmaceutically acceptable salts wherein the release of active ingredient is sustained by release modifying agents which are pH dependent polymers.

Another object of the present invention is related to a sustained release matrix form design comprising acemetacin or its acceptable salt thereof as active ingredient which provides decreased dissolution of acemetacin or a salt form in acidic media (stomach), increased dissolution in basic media (intestine).

A further object of the present invention is to provide a pharmaceutical formulation which sustains therapeutic efficacy due to sustained release of acemetacin by inhibition of its solubility in stomach and allow to release at a specific dissolution rate in the intestine. In this way, it is possible to avoid possible side effects that may occur in stomach.

### Detailed Description of the Invention

Based on the prior art, the object of the invention is related with pharmaceutical formulations comprising an anti-muscarinic agent in a sustained release formulation.

The present invention relates to sustained release pharmaceutical compositions for oral administration comprising non-micronized acemetacin or a pharmaceutically acceptable salt thereof, wherein pharmaceutical composition comprising at least two release modifying agents in a specified ratio between them and in the formulation by weight with a specified API particle size.

The present invention provides a sustained-release oral pharmaceutical composition comprising acemetacin or a pharmaceutically acceptable salt thereof and at least one release modifying agents which permit acemetacine to be released beginning from pH 5.5 and continue through pH 6.0. Thus, the formulation ensures that the active ingredient does not dissolve between pH 5.0, since Eudragit L100-55 provides release from pH 5.5 and Eudragit L100 releases from pH 6.0.

Preferably, pH dependent polymer soluble starting from pH 5.5 is selected from, but are not limited to, hypromellose phthalate, hypromellose acetate succinate, cellulose acetate phthalate, copolymer of methylmethacrylic acid and methyl methacrylate and the mixtures thereof as Eudragit derivatives; more preferably, Eudragit^{®} LI 00-55.

Preferably, pH dependent polymer soluble starting from pH 6.0 is selected from, but not limited to, copolymer of methylmethacrylic acid and methyl methacrylate, cellulose acetate phthalate, cellulose acetate succinate, hydroxypropyl methylcellulose phthalate, hydroxypropyl methylcellulose acetate succinate and the mixtures thereof as Eudragit derivatives; more preferably, Eudragit^{®} L100.

The concentration of the release modifing agent (% weight/total weight), pH values of dissolution, the ratio of the proportions of the combined use of more than one release modifying agent, type and amount of of filler are critical parameters, especially due to the variation in chemical and physical properties of the release modifiers.

The present invention is a sustained release pharmaceutical formulation comprising at least two release modifying modifying agent in ratio of 40:60 to 60:40 by weight, preferably 55:45 to 45:55 by weight, more preferably about 50:50 (1:1) by weight with an amount of between 7% - 15% w/w, preferably 10% - 15%, more preferably about 13% in the composition wherein the particle size of acemetacin is in non-micronized form.

The term used"non-micronised" means that particle size of at least 90% of acemetacin particles in the range of 120 - 160 micrometers.

The term used"micronised" means that particle size of at least 90% of acemetacin particles is less than 7 micrometers.

The invention preferably comprises acemetacin or equivalent salt in an amount ranging from 60 mg to 180 mg, preferably 60 mg to 90 mg, more preferably 90 mg.

The said invention is achieved by combining acemetacin or one of its pharmaceutically acceptable salts with at least one release modifying agent further include one or more of other pharmaceutically acceptable excipients selected from, but not limited to, binder, lubricant, glidant, disintegrant or any combination thereof.

In a preferred embodiment of the invention the filler is selected from the group consisting of lactose, microcrystalline cellulose, starch, pregelatinized starch, modified starch, calcium phosphate (dibasic and / or tribasic), calcium sulfate trihydrate or dihydrate, calcium carbonate, kaolin, cellulose, dextrose, dextrates, dextrin, sucrose, maltose, fructose, glucose, mannitol, maltol, sorbitol, xylitol, titanium dioxide (TiCT). Preferably, the filler is lactose monohydrate or the mixture of lactose monohydrate and titanium dioxide (TiCT).

In a preferred embodiment of the invention, the release modifier is at least one of the group consisting of starch, sugar, ethyl cellulose, cellulose acetate phthalate, hydroxypropylmethyl cellulose acetyl succinate, microcrystalline cellulose, acrylate copolymers (Eudragit derivatives), sodium carboxymethyl cellulose, polyvinyl acetate, hydroxypropylcellulose. Preferably, the release modifier is a mixture of at least two of the methacrylate copolymer derivatives.

The multiple unit dosage form is preferably filled into a soft or hard gelatin capsule or compressed into tablets. In a preferred embodiment of the invention, the dosage form is soft or hard gelatin capsules.

In the preferred embodiment of the invention, oral dosage forms containing multiple units are prepared. The plurality of units may contain the above-mentioned excipients in addition to the active substance. These excpients; one or more excipients selected from the group consisting of binders, lubricants, antistatic agents, dispersants, surface active agents, plasticizers (such as triacetin), glidants.

In a preferred embodiment of the invention, the binder is one of the group comprising gelatin, hydroxypropylcellulose (HPC), hydroxypropylmethylcellulose (HPMC), methyl cellulose, polyvinyl pyrrolidone (PVP), sucrose, starch or the mixture thereof. Preferably, the binder is polyvinyl pyrrolidone.

In a preferred embodiment of the invention, said dosage forms comprise a pharmaceutically acceptable lubricant selected from at least one ofthe group comprising calcium stearate, glycerin, vegetable oil, magnesium stearate, mineral oil, polyethylene glycol, propylene glycol or the mixture thereof.

In a preferred embodiment of the invention said dosage forms comprise a pharmaceutically acceptable lubricant selected from the group consisting of kaolin, talc, silicon dioxide, and the mixture thereof.

In a preferred embodiment of the invention, at least one disintegrant selected from the group comprising alginate, croscarmellose sodium, crospovidone, sodium starch glycollate, pregelatinized starch is used in the production of such dosage forms.

In the preferred embodiment of the invention, at least one glidant selected from the group comprising colloidal silicon dioxide, magnesium stearate, starch, talc, is used in the production of such dosage forms.

The pH dependent polymer may be selected from the group of cellulose acetate, cellulose acetate phthalate, cellulose acetate trimellitate, cellulose acetate succinate, hydroxypropyl methylcellulose (hypromellose) phthalate, hydroxypropyl phthalate, hydroxypropyl methyl cellulose acetate phthalate, hydroxypropyl methylcellulose (hypromellose) acetate succinate, polyvinyl acetate phthalate, shellac, co-polymerized methacrylic acid/methacrylic acid methyl esters such as, materials known under the trade name EUDRAGIT^{®} L12.5, L100, L30D-55, L100-55, S100, S12.5 and derivatives and mixtures thereof.

EUDRAGIT^{®} L100 is described at the Ph.Eur (Methacrylic Acid - Methyl Methacrylate Copolymer (1:1)), USP/NF (Methacrylic Acid Copolymer, Type A - NF) and JP (Methacrylic Acid Copolymer L) monographs. Glass transition temperature (Tg) is above 130°C (+/-5°C).

Chemical/IUPAC name of LI 00 is"Poly(methacylic acid-co-methyl methacrylate) 1 : 1". It dissolves above pH 6.0 and targeted drug release area is jejunum.

EUDRAGIT^{®} L100-55 is described at the Ph.Eur (Methacrylic Acid - Ethyl Acrylate Copolymer (1:1) Type A), USP/NF (Methacrylic Acid Copolymer, Type C - NF) and JP (Dried Methacrylic Acid Copolymer LD) monographs. Glass transition temperature (Tg) is 96°C (+/-5°C).

Chemical/IUPAC name of L100-55 is"Poly (methacylic acid-co-ethyl acrylate) 1 . 1". It dissolves above pH 5.5 and targeted drug release area is duodenum.

Manufacturing method: Acemetacin and the other excipients are screened through a proper sieve and mixed. Then, granulation solution is prepared by dissolving at least one binder in the alcohol. The wet mass is dried and mixed with the release modifying agent solution comprising Eudragit L100 and L100-55 polymers. This mixture is granulated, screened through a sieve and dried. Other excipients used to obtain a final blend are added and the final blend is filled into a proper size of capsule.

### EXAMPLES

Sustained release pharmaceutical composition formulations are tested using the USP apparatus I (basket) at 100 rpm for 180 minutes at 37°C±5°C by using pH 5.5 USP buffer dissolution medium.

One of the most critical parameters of the sustained release formulations is the type and the amount of release modifying agent. According to the acceptable relase profile of acemetacin sustained relase formulations the active ingredient should be absorbed in intestine region which the pH value is higher than 5.5. Based on this information release modifying agent should be selected according to pH values that the polymer is not dissolved under pH 6.0. Eudragit type of release modifying agents are frequently used for this purpose in sustained release formulations. Eudragit L100-55 is active above pH 5.5 and Eudragit L100 is active above pH 6.0 which makes the sustained release formulations available.

Three examples for sustained release formulations are proposed. Upper and lower limits of Eudragit amounts in the composition are investigated.

| **Formulation** | **Eudragit Type (50:50)** | **Eudragit Amount, %** |
|---|---|---|
| Formulation I | Eudragit L100 : Eudragit L100-55 | 6.0 |
| Formulation II | Eudragit L100 : Eudragit L100-55 | 7.0 - 15.0 |
| Formulation III | Eudragit L100 : Eudragit L100-55 | above 15.0 (specified as 21.0) |

The manufacturing method is wet granulation. Eudragit L100 and Eudragit L100-55 in the ratio of 50:50 are used as release modifying agents. There is a matrix composition which presents the sustained release profile.

Dissolution tests are conducted on these formulations and the results are presented in the table below.

**Table 1. Comparative Dissolution Profiles of Formulation I, II, III**

| | **Formulation 1** | **Formulation II** | **Formulation III** |
|---|---|---|---|
| **Time, min.** | **Dissolution in pH 5.5, %** | | |
| **0** | 0.0 | 0.0 | 0.0 |
| **5** | 3.7 | 6.3 | 2.2 |
| **10** | 9.3 | 9.1 | 3.8 |
| **15** | 13.4 | 11.3 | 4.6 |
| **20** | 16.8 | 14.2 | 5.1 |
| **30** | 22.3 | 18.3 | 5.8 |
| **45** | 28.4 | 23.3 | 6.3 |
| **60** | 11.1 | 27.5 | 6.8 |
| **90** | 40.3 | 33.5 | 7.4 |
| **120** | 45.9 | 38.0 | 8.1 |
| **180** | 54.5 | 45.5 | 9.5 |

Dissolution results of Formulation III are very low that the means high amount of release modifying agents in the composition does not permit active substance to be released properly.

Dissolution results of Formulation I is faster than Formulation II and the results to be achieved for the proposed SR formulation.

According to the dissolution results presented above, Formulation II provides better dissolution profile.

In sustained release formulations, the skilled person in the art knows that particle size of active ingredient is also important on in-vitro dissolution profile. To investigate this effect, another in vitro dissolution test is carried out on the formulations prepared by using micronised (<10pm) acemetacine as Formulation IV and non-micronised (125 pm) acemetacine as Formulation V. Quantitative and qualitative compositions are the same as Formulation IF However PSD of the API used are totally different.

| | **Formulation IV** | **Formulation V** |
|---|---|---|
| **Time, min.** | **Dissolution in pH 5.5, %** | |
| **0** | 0.0 | 0.0 |
| **5** | 21.7 | 63 |
| **10** | 33.5 | 9.1 |
| **15** | 36.6 | 11.3 |
| **20** | 39.0 | 14.2 |
| **30** | 41.7 | 1 8.3 |
| **45** | 44.8 | 23.3 |
| **60** | 47.0 | 27.5 |
| **90** | 50.5 | 33.5 |
| **120** | 53.1 | 38.0 |
| **180** | 57.1 | 45.5 |

According to the results presented above, PSD effect of API on sustained-release acemetacin formulations manufactured by following the manufacturing characterizations of Formulation II are as expected as the skilled person proposed. However; regarding the dosage form intended to be sustained-release, the in vitro profile of Formulation V is more proper.

While the invention has been described with respect to the above specific embodiments, it should be recognized that various modifications and changes may be made to the invention by those skilled in the art which also fall within the scope of the invention as defined by the appended claims.

According to the all results, the sustained release pharmaceutical composition comprising acemetacin or a pharmaceutically acceptable salt thereof characterized in that,
- the composition is composed of Eudragit^{®} L100-55 (Poly(methacylic acid-co-ethyl acrylate) and Eudragit^{®} L100 (Poly(methacylic acid-co-methyl acrylate) as release modifying agents in ratio of 40:60 to 60:40 by weight,
- the amount of release modifying agents is between 7% - 15% w/w in the composition,
- the particle size of acemetacin is in the range of 120 - 160 micrometers.

## Claims

1. A sustained release pharmaceutical composition comprising acemetacin or a pharmaceutically acceptable salt thereof wherein
• the composition is composed of Poly(methacylic acid-co-ethyl acrylate) and Poly(methacylic acid-co-methyl acrylate) as release modifying agents in ratio of 40:60 or 60:40 by weight,
• the amount of release modifying agents is between 7% - 15% w/w in the composition,
• the particle size of acemetacin is in the range of 120 - 160 micrometers.

2. A sustained release pharmaceutical composition according to claim. 1, wherein the composition is composed of Poly(m.ethacylic acid-co-ethyl acrylate and Poly(methacylic acid-co-methyl acrylate as release modifying agents in ratio of 55:45 or 45:55 by weight.

3. A sustained release pharmaceutical composition according to claim 2, wherein the composition is composed of Poly(methacylic acid-co-ethyl acrylate) and Poly(methacylic acid-co-methyl acrylate) as release modifying agents in ratio of about 50:50 by weight.

4. A sustained release pharmaceutical composition according to claim 1, wherein the amount of release modifying agents is between 10%-15% w/w in the composition.

5. A sustained release pharmaceutical composition according to claim 1, wherein the amount of release modifying agents is 13% w/w in the composition.

6. A sustained release pharmaceutical composition according to any of the preceding claims wherein the composition also comprises one or more excipients.

7. A sustained release pharmaceutical composition according to claim 1, the pharmaceutical compositon is in the dosage form of soft or hard gelatine capsule comprising tablet, film coated tablet, micro tablet, pellet, powder, granule or combinations thereof.

8. A sustained release pharmaceutical composition according to claim 1, the composition is in the dosage form of hard gelatine capsule comprising more than one granules.

9. A sustained release pharmaceutical composition according to any of the preceding claims wherein the composition comprises 60 mg to 180 mg acemetacin or equivalent amount of therapeutically acceptable salt or the mixture thereof

10. A wet granulation method for preparing sustained release pharmaceutical composition according to any of the preceding claims, wherein the process comprising the step of
• the wet mass comprising acemetacin and at least one pharmaceutically acceptable excipient is dried and mixed with the release modifying agent solution comprising Poly(methacylic acid-co-methyl acrylate) and Poly(methacylic acid-co-ethyl acrylate) polymers.

11. A sustained release pharmaceutical composition according to any of the preceding claims for use in the treatment of osteoarthritis, rheumatoid arthritis and ankylosing spondylitis, and acute gouty arthritis, acute musculoskeletal pain, postoperative pain, and dysmenorrhea.

## Patentansprüche

1. Eine pharmazeutische Zusammensetzung mit verzögerter Freisetzung, bestehend aus Acemetacin oder ein pharmazeutisch erträgliches/zulässiges Salz,
• indem die Zusammensetzung aus Poly(methacylsäure-Co-Ethylacrylat) und Poly(Methacylsäure-Co-Methylacrylat) als freisetzungsmodifizierendes Wirkstoff in einem Gewichtsverhältnis von 40:60 oder 60:40 besteht,
• die Menge an freisetzungsmodifizierenden Wirkstoff zwischen 7 % - 15 % (w/w) in der Zusammensetzung befindet u.
• die Partikel von Acemetacin im Bereich von 100 - 200 Mikrometern liegen.

2. Eine pharmazeutische Zusammensetzung mit verzögerter Freisetzung nach Anspruch 1, wobei nicht-mikronisierte Acemetacin-Partikel eine Partikelgröße im Bereich von 120-160 µm aufweisen.

3. Eine pharmazeutische Zusammensetzung mit verzögerter Freisetzung nach Anspruch 1, wobei die Zusammensetzung aus Poly(Methacylsäure-Co-Ethylacrylat) und Poly(Methacylsäure-Co-Methylacrylat) als freisetzungsmodifizierendes Wirkstoff in einem Gewichtsverhältnis von 55:45 oder 45:55 besteht.

4. Eine pharmazeutische Zusammensetzung mit verzögerter Freisetzung nach Anspruch 3, wobei die Zusammensetzung aus Poly(Methacylsäure-Co-Ethylacrylat) und Poly(Methacylsäure-Co-Methylacrylat) als freisetzungsmodifizierendes Wirkstoff in einem Gewichtsverhältnis von 50:50 besteht.

5. Eine pharmazeutische Zusammensetzung mit verzögerter Freisetzung nach Anspruch 1, wobei die Menge an freisetzungsmodifizierenden Wirkstoff zwischen 10 % - 15 % w/w in der Zusammensetzung befindet.

6. Eine pharmazeutische Zusammensetzung mit verzögerter Freisetzung nach Anspruch 1, wobei die Menge an freisetzungsmodifizierenden Wirkstoff zwischen 13 % w/w in der Zusammensetzung befindet.

7. Eine pharmazeutische Zusammensetzung mit verzögerter Freisetzung nach einem der vorstehenden Ansprüche, wobei die Zusammensetzung auch einen oder mehrere Hilfsstoffe enthält.

8. Eine pharmazeutische Zusammensetzung mit verzögerter Freisetzung nach Anspruch 1, wobei die pharmazeutische Zusammensetzung in der Darreichungsform einer weichen oder harten Gelatinekapsel; bestehend aus Tablette, Filmtablette, Mikrotablette, Pellet, Pulver, Granulat oder Kombinationen daraus.

9. Eine pharmazeutische Zusammensetzung mit verzögerter Freisetzung nach Anspruch 1, wobei die Zusammensetzung in der Darreichungsform einer harten Gelatinekapsel, von mehr aus als ein Granulat besteht.

10. Eine pharmazeutische Zusammensetzung mit verzögerter Freisetzung nach einem der vorstehenden Ansprüche, wobei die Zusammensetzung 60 mg bis 180 mg Acemetacin oder eine äquivalente Menge eines therapeutisch erträglichen/zulässigen Salzes oder einer Mischung daraus besteht.

11. Eine pharmazeutische Zusammensetzung mit verzögerter Freisetzung nach einem der vorstehenden Ansprüche, wobei diese bei der Behandlung von Osteoarthritis, rheumatoider Arthritis und ankylosierender Spondylitis u. akuter Gichtarthritis, akuten muskuloskelettalen Schmerzen, postoperativen Schmerzen und Dysmenorrhö hingewiesen ist.

## Revendications

1. Composition pharmaceutique à libération prolongée comprenant de l'acémétacine ou un sel pharmaceutiquement acceptable de celle-ci, dans laquelle
• la composition est composée de Poly(acide méthacylique- co-éthyl acrylate) et de Poly(acide méthacylique- co-méthyl acrylate) comme agents modificateurs de libération dans un rapport de 40:60 ou 60:40 en poids,
• la quantité d'agents modificateurs de libération est comprise entre 7 % et 15 % p/p dans la composition,
• la taille des particules d'acémétacine est comprise entre 100 et 200 micromètres.

2. Composition pharmaceutique à libération prolongée selon la revendication 1, dans laquelle les particules d'acémétacine non micronisées ont une taille de particule dans la gamme de 120 -160 µm.

3. Composition pharmaceutique à libération prolongée selon la revendication 1, dans laquelle la composition est composée de poly(acide méthacrylique- co-éthyl acrylate) et de poly(acide méthacrylique-co-méthyl acrylate) comme agents modificateurs de libération dans un rapport de 55:45 ou 45:55 en poids.

4. Composition pharmaceutique à libération prolongée selon la revendication 3, dans laquelle la composition est composée de Poly(acide méthacylique- co-éthyl acrylate) et de Poly(acide méthacylique-co-méthyl acrylate) comme agents modificateurs de libération dans un rapport d'environ 50:50 en poids.

5. Composition pharmaceutique à libération prolongée selon la revendication 1, dans laquelle la quantité d'agents modificateurs de libération est comprise entre 10 % et 15 % p/p dans la composition.

6. Composition pharmaceutique à libération prolongée selon la revendication 1, dans laquelle la quantité d'agents modificateurs de libération est de 13% p/p dans la composition.

7. Composition pharmaceutique à libération prolongée selon l'une quelconque des revendications précédentes, dans laquelle la composition comprend également un ou plusieurs excipients.

8. Composition pharmaceutique à libération prolongée selon la revendication 1, la composition pharmaceutique est sous la forme posologique d'une capsule de gélatine molle ou dure comprenant un comprimé, un comprimé pelliculé, un micro-comprimé, une pastille, une poudre, un granule ou des combinaisons de ceux-ci.

9. Composition pharmaceutique à libération prolongée selon la revendication 1, la composition est sous la forme posologique d'une capsule de gélatine dure comprenant plus d'un granule.

10. Composition pharmaceutique à libération prolongée selon l'une quelconque des revendications précédentes, dans laquelle la composition comprend 60 mg à 180 mg d'acémétacine ou une quantité équivalente de sel thérapeutiquement acceptable ou leur mélange.

11. Composition pharmaceutique à libération prolongée selon l'une quelconque des revendications précédentes, dans laquelle elle est indiquée dans le traitement de l'arthrose, de la polyarthrite rhumatoïde et de la spondylarthrite ankylosante, et de l'arthrite goutteuse aiguë, des douleurs musculo-squelettiques aiguës, des douleurs postopératoires et de la dysménorrhée.
